# EUROPEAN PATENT APPLICATION

(11) **EP 4 102 441 A1**
(43) Date of publication of application: **14.12.2022**
(21) Application number: 21761865.1
(22) Date of filing: 26.02.2021
(51) Int. Cl.: G06Q 50/02, A01K 29/00

(54) **LIVESTOCK RAISING INFORMATION MANAGEMENT SYSTEM, LIVESTOCK RAISING INFORMATION MANAGEMENT SERVER, LIVESTOCK RAISING INFORMATION MANAGEMENT METHOD, AND LIVESTOCK RAISING INFORMATION MANAGEMENT PROGRAM**

(30) Priority: 27.02.2020 JP 2020032373
(71) Applicant: Eco-Pork Co., Ltd., Tokyo, 130-0003 (JP)
(72) Inventor: KAMBAYASHI, Takashi, Tokyo 130-0003 (JP); MAKINO, Tsuyoshi, Tokyo 130-0003 (JP); MAGATA, Atsushi, Tokyo 107-6024 (JP); FUJISAWA, Ippei, Tokyo 107-6024 (JP); NAKAYAMA, Tomohiro, Tokyo 107-6024 (JP)
(74) Representative: Fédit-Loriot
(86) International application number: PCT/JP2021/007582
(87) International publication number: WO 2021/172574

(57) **Abstract**

A livestock information management system 1 includes: a livestock image information acquisition unit 112 configured to acquire livestock image information including a plurality of livestock belonging to a management herd; a feeding information acquisition unit 114 configured to acquire feeding information on feed for the livestock belonging to the management herd; a herd weight estimation unit 116 configured to estimate a herd weight distribution of the plurality of livestock from the livestock image information; and a livestock information management unit 118 configured to generate livestock management information by associating the herd weight distribution estimated by the herd weight estimation unit with the feeding information.

## Description

### TECHNICAL FIELD

The present disclosure relates to a livestock information management system, a livestock information management server, a livestock information management method, and a livestock information management program.

### BACKGROUND ART

In the livestock industry which produces meat distributed via ranking of meat, it is required to bring individual weights of livestock to be in a weight range defined as an upper grade, in a shorter time in order to maximize the profits of livestock farmers. For this reason, the livestock farmers change the contents of feed with the increase in weight of livestock, and particularly change the contents of feed to adjust the quality of meat before shipping. Therefore, the livestock farmers timely measure weights of individual livestock, and adjust the type and amount of feed and the like based on the measured weights.

For example, in Patent Document 1, imaging devices are installed at boundaries between a rest area provided in a raising place and multiple feeding areas where different types of feed are placed, and image data on individual animals are acquired by the imaging devices when raising animals (livestock) pass through the boundaries to automatically estimate weights of the individual animals. Then, the weight data acquired is compared with an evaluation reference weight set in advance to guide and distribute a herd of individual animals in the rest area to different feeding areas.

### CITATION LIST

### PATENT DOCUMENT

PATENT DOCUMENT 1: Japanese Unexamined Patent Publication No. 2007-175050

### SUMMARY OF THE INVENTION

### TECHNICAL PROBLEM

However, in the technology described in Patent Document 1, it is necessary to first define the rest area and the multiple feeding areas and install the imaging devices at the boundaries. There is thus a problem in that it is necessary to secure a space and a large-scale facility, and the facility cost increases in accordance with the number of devices installed. In addition, in order to guide the livestock to the boundaries, a guiding work by a farmer, training of the livestock, and the like are required, which leads to an increase in cost thereof, and burdens on the farmer and the livestock become large.

In addition, when a large amount of livestock is reared by a small number of workers, it is more efficient to manage the livestock as a group (herd) for even changing the feed. For the management, it is effective to grasp a weight distribution which is a weight state as a group. However, in the technology described in Patent Document 1, the weight is estimated for each animal at the boundary, and the feeding area is assigned according to the estimated weight. Thus, as the number of livestock to be reared increases, a longer time is required for the estimation, or costs increases due to installation of a large number of devices. Accordingly, it is not suitable for rearing management in a large-scale rearing farm with a small number of workers.

In order to solve such a problem, the present disclosure is intended to provide a livestock information management system, a livestock information management server, a livestock information management method, and a livestock information management program which can easily manage weights and feed for livestock while reducing the increase in cost even in a large-scale rearing farm with a small number of workers.

### SOLUTION TO THE PROBLEM

In order to achieve the objective, a livestock information management system according to the present disclosure includes: a livestock image information acquisition unit configured to acquire livestock image information including a plurality of livestock; a feeding information acquisition unit configured to acquire feeding information on feed for the livestock; a herd weight estimation unit configured to estimate a herd weight distribution of the plurality of livestock from the livestock image information; and a livestock information management unit configured to generate livestock management information by associating the herd weight distribution estimated by the herd weight estimation unit with the feeding information.

In the livestock information management system, the livestock information management unit may generate the livestock management information by associating the weight distribution with the feeding information so that an imaging timing for the livestock image information on which the herd weight distribution is based corresponds to a feeding timing in the feeding information.

In the livestock information management system, the livestock information management unit may be configured to generate trend information along a time series of the herd weight distribution and the feeding information associated with the herd weight distribution.

In the livestock information management system, the feeding information may include at least one of a feeding timing, the type of feed, a feeding amount, a nutrient, an additive, a water supply timing, or a water supply amount.

In the livestock information management system, the herd weight estimation unit may identify regions indicating individuals of the livestock in the livestock image information, calculate estimated weights corresponding to the regions of the individuals, and estimate the herd weight distribution from the estimated weights.

In the livestock information management system, the herd weight estimation unit may identify predetermined parts of the livestock from the regions of the individuals identified, and exclude an individual which does not include one or more specific parts necessary for weight estimation among the parts identified, from a target for estimation of the herd weight distribution.

In the livestock information management system, the herd weight estimation unit may identify predetermined parts of the livestock from the regions of the individuals identified, and calculate an estimated weight of an individual including one or more specific parts necessary for weight estimation among the parts identified by complementing a missing part, if any, for an outline of the livestock in the region of the individual identified.

In the livestock information management system, the herd weight estimation unit may exclude an individual where a value correlated with the size of the region of the individual identified is less than a predetermined threshold, from a target for estimation of the herd weight distribution.

In the livestock information management system, for an individual where the value correlated with the size of the region of the individual identified is equal to or higher than the predetermined threshold, the herd weight estimation unit may calculate an estimated weight of the individual by complementing of a missing part, if any, for an outline of the livestock in the region of the individual identified.

In the livestock information management system, the herd weight estimation unit may determine postures of the individuals identified and calculate estimated weights of the individuals by using an estimation model corresponding to each of the postures.

In the livestock information management system, the livestock information management unit may have an abnormality determination condition for determining an abnormal individual from the herd weight distribution, and generate alert information when the herd weight distribution estimated by the herd weight estimation unit satisfies the abnormality determination condition.

In the livestock information management system, the livestock information management unit may generate alert information when the livestock image information acquisition unit cannot acquire the livestock image information normally or the feeding information acquisition unit cannot acquire the feeding information normally.

In order to achieve the obj ective, a livestock information management server according to the present disclosure includes: a livestock image information acquisition unit configured to acquire livestock image information including a plurality of livestock; a feeding information acquisition unit configured to acquire feeding information on feed for the livestock; a herd weight estimation unit configured to estimate a herd weight distribution of the plurality of livestock from the livestock image information; and a livestock information management unit configured to generate livestock management information by associating the herd weight distribution estimated by the herd weight estimation unit with the feeding information.

In order to achieve the objective, a livestock information management method according to the present disclosure includes: acquiring, by a livestock image information acquisition unit, livestock image information including a plurality of livestock; acquiring, by a feeding information acquisition unit, feeding information on feed for the livestock; estimating, by a herd weight estimation unit, a herd weight distribution of the plurality of livestock from the livestock image information; and generating, by a livestock information management unit, livestock management information by associating the herd weight distribution estimated in the estimating of the herd weight distribution with the feeding information.

In order to achieve the objective, a livestock information management program according to the present disclosure causes a computer to execute: acquiring, by a livestock image information acquisition unit, livestock image information including a plurality of livestock; acquiring, by a feeding information acquisition unit, feeding information on feed for the livestock; estimating, by a herd weight estimation unit, a herd weight distribution of the plurality of livestock from the livestock image information; and generating, by a livestock information management unit, livestock management information by associating the herd weight distribution estimated in the estimating of the herd weight distribution with the feeding information.

### ADVANTAGES OF THE INVENTION

According to the present disclosure using the above-described means, the weights and feed for the livestock can be easily managed while reducing the increase in cost.

### BRIEF DESCRIPTION OF THE DRAWINGS

FIG. 1 is a schematic block diagram illustrating a configuration of a livestock information management system according to an embodiment of the present disclosure.
FIG. 2 is a schematic view illustrating an example of the inside of a barn.
FIG. 3 is an example of information stored in each database of a livestock information management server.
FIG. 4 is an example of a livestock image.
FIG. 5 is an example of a result of individual identification processing on the livestock image of FIG. 4.
FIG. 6 is an explanatory diagram of representative point identification.
FIG. 7 is a graph illustrating trends of a herd weight distribution and a feeding amount associated with each other.
FIG. 8 is part of a flowchart showing a livestock management information generation routine executed by the livestock information management server of the livestock information management system according to the embodiment of the present disclosure.
FIG. 9 is a flowchart subsequent to FIG. 8.
FIG. 10 is a schematic block diagram illustrating a configuration of a computer according to the embodiment.

### DESCRIPTION OF EMBODIMENTS

Embodiments of the present disclosure will now be described with reference to the drawings.

In the embodiments of the present disclosure described below, pigs are mainly taken as an example of livestock. In the livestock industry, the number of pigs reared in a single farm may range from several thousands to several tens of thousands, and the burden of managing such an enormous number of livestock is large.

Pigs are mainly divided into breeding pigs and non-breeding pigs. Breeding pigs are pigs reared to give birth to piglets. Non-breeding pigs are pigs (porkers) which are reared to be shipped after growth. The number of non-breeding pigs is larger than that of breeding pigs; thus, it is difficult to manage each individual non-breeding pig and is common to manage multiple pigs as a herd. The pigs described below are mainly intended for non-breeding pigs (porkers). In the following description, the terms "age in day," "age in week," and "age in month" refer to the number of days, the number of weeks, and the number of months elapsed from a weaning date as a reference date, respectively. The age in day may also be the number of days elapsed from a birth date as a reference date.

The selling price of pigs for distribution in Japan is determined, in each of central wholesale markets and local meat markets, by multiplying a ranking or grade unit which is determined based on the weight and meat quality of individual pigs supplied by a wholesale price (unit price per kg), while taking a demand-supply relationship into account. The grades are determined based on whether or not the half-carcass weight of the slaughtered dressed carcass pork is within defined ranges as a prerequisite. The grades are classified into four: "prime," "high," "medium," and "regular," and further classified into "out of grades" which does not correspond to any grade, in accordance with conditions for the application of the trading standards defining meat and the like. Therefore, in order to increase profits, it is desirable to rear pigs so that their weights fall within the ranges of the grades (appropriate weight range). Further, in order to obtain a higher grade of "high" or "prime," at least the weights are made in appropriate weight ranges. Accordingly, further increase in the profitability can be expected.

It is common for pigs to be reared in multiple divided pig pens in a barn, which is a building structure in a farm. Each pig pen is an environment in which several tens of pigs are reared. In addition, several tens of pigs put into a pig pen are treated as a management herd.

### <Configuration>

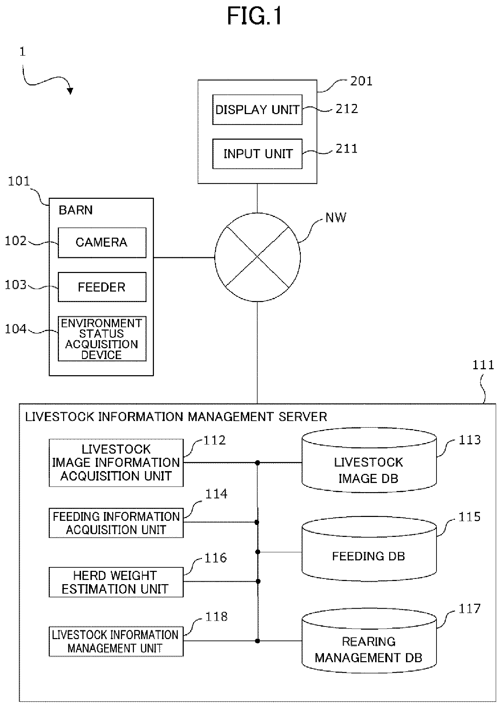

FIG. 1 is a system configuration diagram illustrating a livestock information management system 1 including a livestock information management server 111 according to an embodiment of the present disclosure. The present embodiment will be described below assuming that the livestock to be managed are pigs, particularly porkers to be herd-managed. As shown in FIG. 1, in the livestock information management system 1 according to the embodiment of the present disclosure, the livestock information management server 111 is communicably connected with a barn and a pig pen (hereinafter referred to as a barn) 101 where pigs are reared and an information terminal 201 of a farmer via the network NW such as the Internet, a LAN, and a virtual private network (VPN). For simplification of description, FIG. 1 illustrates only one barn 101 and one information terminal 201, but the livestock information management server 111 is connectable to multiple barns 101 and multiple information terminals 201 via the network NW. The livestock information management server 111 may be a so-called cloud server in cloud computing.

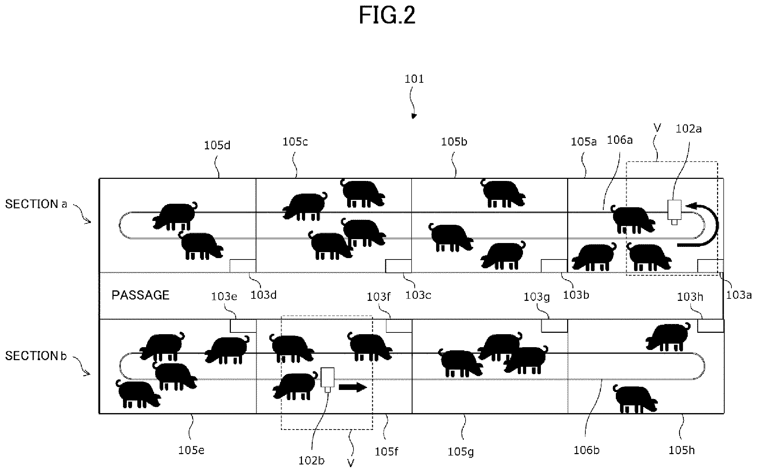

FIG. 2 is a schematic diagram illustrating an example of the inside of the barn 101 in the present embodiment, and the barn 101 will be described in detail below with reference to FIGS. 1 and 2.

As shown in FIG. 2, the inside of the barn 101 is, for example, divided into two sections a and b with a central passage interposed therebetween, and in the sections a and b, pig pens 105a to 105d and the pig pens 105e to 105h (hereinafter collectively referred to as the pig pens 105) are formed side by side in a line. Several to several tens of porkers are reared in each pig pen 105, and one pig pen 105 is managed as one management herd.

In addition, in the barn 101, rails 106a and 106b are provided on the respective ceilings of the sections a and b so as to extend over the pig pens 105a to 105d and the pig pens 105e to 105h. Cameras 102a and 102b (hereinafter collectively referred to as cameras 102) are provided on the rails 106a and 106b so as to be movable along the rails 106a and 106b.

The cameras 102 are, for example, visible light cameras and each have a function of detecting light reflected from an object and generating image (still image or moving image) information. As each camera 102, an infrared camera and an infrared light may be used in combination in addition to the visible light camera, so as to be able to perform nighttime imaging.

The cameras 102 in the present embodiment are directed downward, and capture one or multiple images of a certain imaging area V of each pig pen 105 at a regular basis as indicated by a dotted line in FIG. 2 while moving along the rails 106a and 106b. The cameras 102 can generate livestock image information including images of livestock in the pig pens 105, imaging timing information, and position information. The imaging timing includes information on year, month, day, and time. Further, the position information corresponds to each pig pen 105, and which pig pen 105 the livestock image is captured can be determined from the position information.

In the barn 101, the pig pens 105a to 104h are provided with feeders 103a to 105h (hereinafter also collectively referred to as feeders 103), respectively. Each feeder 103 is a device for providing feed and water to pigs. The feeder 103 can automatically supply the predetermined amounts of predetermined kind of feed and water at timings determined for each pig pen 105. The feeder 103 can generate feeding information including information on the type and amount of feed (feeding amount) and the amount of water (water supply amount) which have been supplied to each pig pen 105, the supply timing, and the like.

Although not shown in FIG. 2, the barn 101 is further provided with an environment status acquisition device 104 configured to acquire environment status information such as a temperature and a humidity, an air flow rate, a light quantity, odor, and sound. Specifically, the environment status acquisition device 104 is a sensor unit in which a temperature-humidity sensor for detecting a temperature and a humidity in the barn 101, an air flow rate sensor for detecting an air flow rate in the barn 101, a light quantity sensor for detecting a light quantity in the barn 101, an odor sensor for detecting odor in the barn 101, a sound collector (microphone) for detecting sound in the barn 101, and the like are integrated. The environment status acquisition device 104 may be provided in each pig pen 105, and in this case, the environment status information on each pig pen 105 can be detected.

The barn 101 can transmit, at a regular basis, the livestock image information generated by the cameras 102, the feeding information generated by the feeders 103, and the environment status information acquired by the environment status acquisition device 104 to the livestock information management server 111 via the network NW. These pieces of information on the barn 101 are not necessarily generated by the cameras 102, the feeders 103, and the environment status acquisition device 104 themselves, and may be generated via a computer such as an information terminal provided in the barn 101 based on pieces of information generated by the cameras 102, the feeders 103, and the environment status acquisition device 104. A barn ID and a pig pen ID for identifying a target barn and a target pig pen have been assigned to the pieces of information transmitted from the barn 101.

The livestock information management server 111 includes one or more servers (computers) that execute processes based on programs, which have various computation units and storages. The livestock information management server 111 functionally mainly includes a livestock image information acquisition unit 112, a livestock image database 113, a feeding information acquisition unit 114, a feeding database 115, a herd weight estimation unit 116, a rearing management database 117, and a livestock information management unit 118 (hereinafter, each database is referred to as DB).

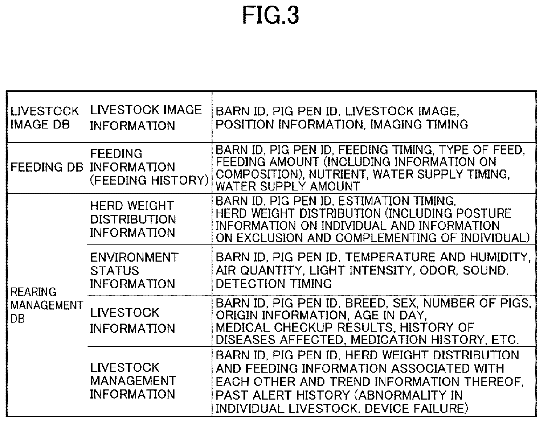

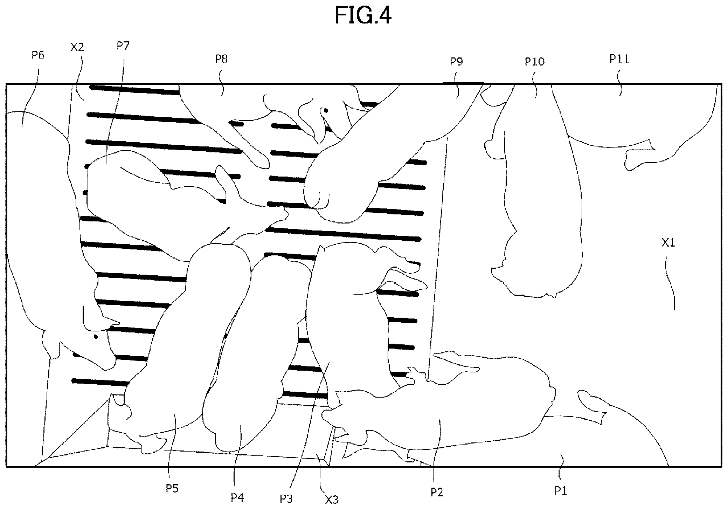

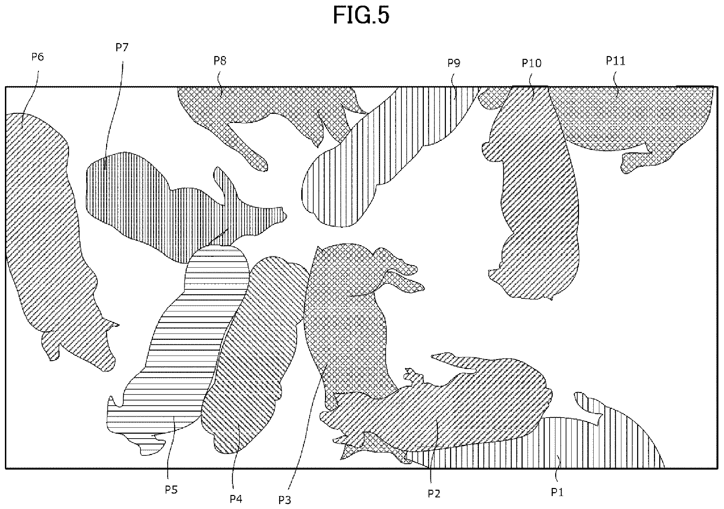

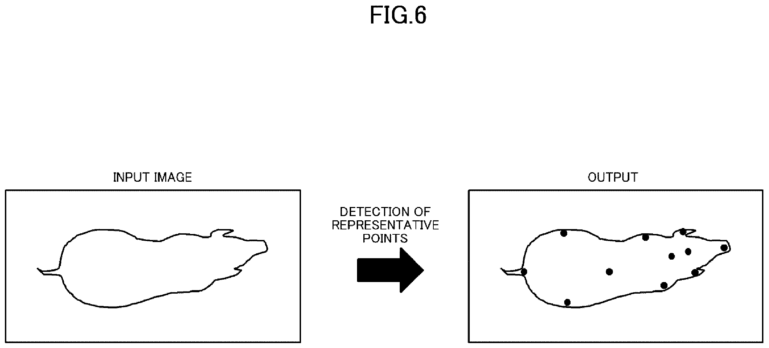

FIG. 3 shows an example of information stored in each of DBs 113, 115, and 117. FIG. 4 shows an example of a livestock image. FIG. 5 shows an example of a result of individual identification processing performed on the livestock image of FIG. 4. FIG. 6 is an explanatory diagram of representative point identification. The detailed configuration of the livestock information management server 111 will be described below with reference to FIGS. 1, 3, and 6.

The livestock image information acquisition unit 112 has a function of acquiring livestock image information generated by the cameras 102 in the barn 101 and storing the livestock image information in the livestock image DB 113. As shown in FIG. 3, the livestock image DB 113 stores, as the livestock image information, information such as a barn ID and a pig pen ID corresponding to the livestock image, imaging position information, and an imaging timing in addition to the livestock image. Images of one pig pen 105 obtained by continuously imaging multiple times, i.e., pieces of livestock image information obtained at close imaging timings which are substantially the same are associated with each other as a single image information group and stored.

The feeding information acquisition unit 114 has a function of acquiring feeding information generated by the feeders 103 of the barn 101 and storing the feeding information in the feeding DB 115. As shown in FIG. 3, the feeding DB 115 stores, as the feeding information, a barn ID and a pig pen ID which are targets of feeding, a feeding timing, the type of feed, a feeding amount, a water supply timing, a water supply amount, and the like. In addition, in a case where multiple types of feed are mixed, information on an amount or a mixing ratio for each type of feed is also included as the feeding amount. The feeding information may further include nutrient information and additive information on the feed.

The herd weight estimation unit 116 has a function of estimating a herd weight distribution of multiple pigs reared in the pig pen 105 from the livestock image information. In the estimation of the herd weight distribution, regions indicating individual pigs are identified from the livestock image, estimated weights corresponding to the respective regions of the individuals are calculated, and these estimated weights are subjected to statistical processing, thereby estimating the herd weight distribution.

Specifically, the herd weight estimation unit 116 acquires livestock image information on the pig pen 105 which is a target for estimation from the livestock image DB 113, and identifies the regions indicating individual pigs from the livestock image by image processing. For example, when the acquired livestock image is a livestock image as shown in FIG. 4, not only pigs P1 to P11, but also a floor X1 of the pig pen 105, a feed box X3 and a drainboard X2 installed in the pig pen 105, and the like are reflected in the image. In contrast, the herd weight estimation unit 116 identifies individual pigs by image analysis, so that regions indicating individual pigs P1 to P11 are identified as shown by hatching in FIG. 5, and objects other than the pigs are excluded. By using livestock images captured at substantially the same timing, three-dimensional analysis considering depth information can be performed, and individual identification including a height difference can be performed. Therefore, for example, it is possible to determine that the pig P2 is positioned on the uppermost side among the vertically overlapping pigs P1, P2, and P3 shown in FIGS. 4 and 5.

The herd weight estimation unit 116 then calculates estimated weights of the regions indicating individual pigs P1 to P11 identified as shown in FIG. 5. Specifically, the herd weight estimation unit 116 calculates a value correlated with the size of the region indicating each individual. For example, as the value correlated with the size of the region, the area or the number of pixels of the region is used. The herd weight estimation unit 116 has a computational expression for calculating the estimated weights of pigs in accordance with the areas or the numbers of pixels on the livestock image in advance, and calculates the estimated weights using the computational expression. The computational expression is set to be capable of calculating estimated weights in accordance with the degrees of growth (e.g., ages in day) of the pigs, for example. The computational expression is not necessarily set in accordance with the degrees of growth. In addition, instead of the computational expression, artificial intelligence (AI) for weight calculation where calculation of estimated weights from values correlated with the sizes of the individual regions is machine-learned (e.g., deep neural network) may be used.

The herd weight estimation unit 116 has a function of performing determination of postures of pigs or processing of excluding or complementing of individuals with parts (missing parts) which are not reflected in the livestock image in order to improve accuracy of herd weight estimation.

Specifically, the herd weight estimation unit 116 identifies points (hereinafter, referred to as representative points) representing respective parts of pig for each individual identified. For example, as shown in FIG. 6, the representative points are set to main parts of pig such as the head, ears, nose, eyes, forelimbs, hindlimbs, shoulders, joints, back, abdomen, buttocks, and tail of pig. As each part set as a representative point, a point which is independent of the age in day of pig is selected. The identification of the representative points is performed by an image analysis technique using a learned model, such as pattern matching and deep learning including three-dimensional information, for example.

The herd weight estimation unit 116 determines a posture of each individual from arrangement of the representative points identified for the individual and generates posture information on the individual. As the posture information, information on arrangement of the representative points itself may be used. The posture information includes, for example, standing state, a sitting state, and a lying state. The herd weight estimation unit 116 calculates the estimated weight by using the estimation model corresponding to the posture information.

The herd weight estimation unit 116 determines whether or not each individual is included in a target for herd weight estimation, based on the representative points identified for each individual. Specifically, the herd weight estimation unit 116 makes the determination such that an individual indicated by the identified representative points and not including one or more specific parts necessary for estimated weight calculation is excluded from a target for herd weight distribution estimation and an individual including all specific parts is included as a target for herd weight distribution estimation. One or more specific parts necessary for estimated weight calculation are set to parts having large volume such as the back, the abdomen, and the buttocks, i.e., parts having large proportion in weight.

The herd weight estimation unit 116 can also determine whether or not the individual is included as a target for herd weight distribution estimation based on the value (the area or the number of pixels) correlated with the size of the region of the individual on the livestock image. Specifically, the herd weight estimation unit 116 makes the determination such that an individual where the area or the number of pixels of the region of the individual is less than a predetermined threshold (predetermined area, the predetermined number of pixels) is excluded from a target for herd weight distribution estimation and an individual where the area or the number of pixels of the region of the individual is equal to or higher than the predetermined threshold is included as a target for herd weight distribution estimation. The predetermined threshold is a variable, and is set to be larger in accordance with the degree of growth of the pig such as age in day, for example.

If there is a missing part for an outline of pig in the identified region of the individual, the herd weight estimation unit 116 complements the missing part, and then calculates an estimated weight of the individual included as a target for herd weight distribution estimation. For the complementing, for example, the herd weight estimation unit 116 has part-specific templates in advance, and complements the missing part by using the part-specific template corresponding to the missing part. The part-specific templates are prepared in accordance with the posture and the degree of growth of the pig. A specific technique for the complementing is not limited to this technique, and for example, the complementing may be performed using, for example, AI for complementing obtained by machine learning (e.g., a deep neural network) of the outline of the pig. For example, whether or not the complementing is performed may be determined in accordance with the part or the size of the missing part, such that the complementing is not necessary for a part which has a low influence on the weight, for example.

The herd weight estimation unit 116 estimates the herd weight distribution by statistical processing upon calculation of the estimated weights of all individuals which are targets for the herd weight distribution. For example, the herd weight distribution can be shown as a frequency distribution (histogram) indicating the number of pigs per weight. The estimated herd weight distribution is stored in the rearing management DB 117 as herd weight distribution information including corresponding barn ID, pig pen ID, estimation timing, and the like. The herd weight distribution information further includes, for example, posture information on each individual used for calculation of the estimated weight of each individual and information on the individual excluded and the individual for which the missing part is complemented.

As shown in FIG. 3, the rearing management DB 117 stores, in addition to the herd weight distribution information, environment status information generated by the environment status acquisition device 104 in the barn 101, livestock information, and livestock management information. As the environment status information, information on a barn ID and a pig pen ID, information on a temperature and a humidity detected, an air quantity, a light quantity, odor, and sound, and information on timings when these environment statuses are detected is stored. As the livestock information, information on a barn ID, a pig pen ID, a breed of porkers for each pig pen, a sex, the number of pigs, an origin, an age in day, medical checkup results, a history of diseases affected, a medication history, and the like is stored. As the livestock management information, a barn ID and a pig pen ID, the herd weight distribution and the feeding information associated with each other in the livestock information management unit 118 to be described later, trend information thereof, a past alert history, and the like are stored.

The livestock information management unit 118 has a function of generating livestock management information by associating the herd weight distribution estimated by the herd weight estimation unit 116 with the feeding information stored in the feeding DB 115. The association between the herd weight distribution and the feeding information is performed so that the imaging timing of the livestock image information on which the herd weight distribution is based corresponds to the feeding timing in the feeding information. The correspondence between the imaging timing and the feeding timing can be arbitrarily set by the farmer, and for example, the feeding timing (the timing when feed is supplied by the feeder 103) is associated with the livestock image information acquired at the same imaging timing as the feeding timing, and the feeding timing is associated with the livestock image information at an imaging timing which is after a predetermined period (e.g., 24 hours) from the feeding time until the weight is reflected.

The livestock information management unit 118 can generate, as the livestock management information, trend information along a time series of the herd weight distribution and the feeding information associated with the herd weight distribution.

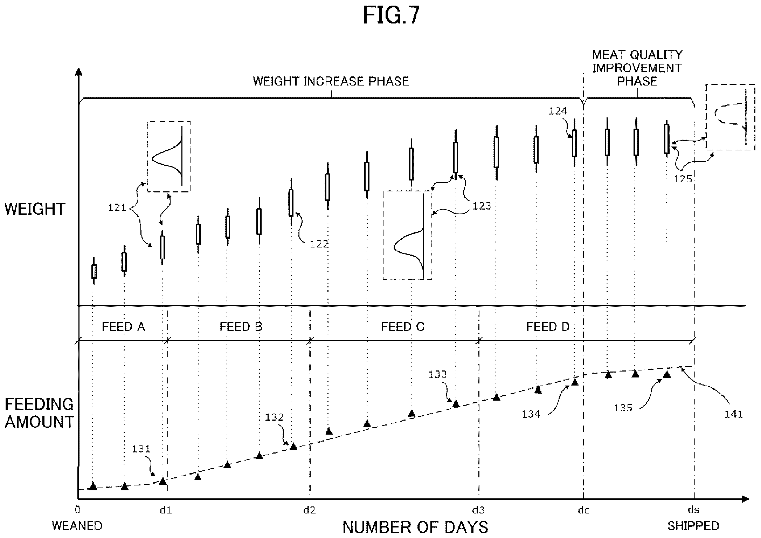

FIG. 7 shows an example of a trend graph where the herd weight distribution and the feeding information are associated with each other. In the trend graph of FIG. 7, the upper graph indicates the trend of the herd weight distribution, and the lower graph indicates the trend of the feeding information. In FIG. 7, the horizontal axis represents the number of days (age in day) elapsed from a weaning date from mother pigs or the like (e.g., a nursing unit), the vertical axis of the upper graph represents the weight, and the vertical axis of the lower graph represents the feeding amount. In FIG. 7, in order to simplify the description, plots of the herd weight distribution and the feeding information are shown at certain intervals in accordance with the number of days (the horizontal axis), but actually, for example, the herd weight distribution and the feeding information are acquired every day or more frequently.

As shown in the upper graph of FIG. 7, porkers (non-breeding pigs) are shipped through a weight increase phase and a meat quality improvement phase. The weight increase phase is a phase for increasing the weights in a predetermined period after weaning. The meat quality improvement phase is a phase for reducing excessive weight increase after the predetermined period and converging the variation in the individual weights of the pigs so that the weight distribution falls within a predetermined range. The period from the weaning date to the age dc in day is the weight increase phase, and the period from the age dc in day to the age ds in day which is the shipping age in day is the meat quality improvement phase. The pigs are herd-managed as mentioned above. Thus, the weights of the pigs belonging to the management herd are represented as a herd weight distribution. In FIG. 7, the herd weight distribution shows a trend in the weight distributions of the pigs belonging to the management herd according to the age in day by box plots. The graph is generated after the estimated age in day in the herd weight distribution 125 and before the age ds in day. Box plots indicated by solid lines show herd weight distributions estimated by the herd weight estimation unit 116 according to the present embodiment, and conceptual diagrams of the frequency distributions representing the herd weight distributions indicated by reference numerals 121, 123, and 125 are also shown besides the corresponding herd weight distributions.

As shown in the lower graph of FIG. 7, feed for the pigs is changed for each raising phase determined mainly based on their weights. Pigs are fed with feed A in a period from the weaning date to the age d1 in day, feed B in a period from the age d1 in day to the age d2 in day, feed C in a period from the age d2 in day to the age d3 in day, and feed D in a period from the age d3 in day to the age ds in day (shipping date). For the feeding amount, based on the rearing standard indicated by the rearing standard line 141 of FIG. 7, the feed is supplied at the ratio of about 6% to about 8% for piglets, about 4% to about 5% for pigs in the growing phase, and about 3.6% to about 4% for pigs in the fattening phase, relative to their weights. The actual measurement values of the feeding amount supplied to pigs herd-managed as a herd are plotted with triangular symbols in the lower graph of FIG. 7. The actual values of the feeding amount are associated with the herd weight distribution, and pieces of information thereof are handled in cooperation with each other. Although not shown in the drawings, a graph of the water supply amount can also be generated in the same manner.

The livestock information management unit 118 may additionally associate environment status information in addition to the herd weight distribution and the feeding information. For example, information on a temperature and a humidity, an air quantity, a light quantity, odor, sound, and the like in the target pig pen at each timing is also indicated in correspondence with the number of days in the trend graph of FIG. 7, so that influence of the environment status on the herd weight distribution can be recognized.

The livestock information management unit 118 has an abnormality determination condition for determining an abnormal individual from the herd weight distribution, and can generate alert information when the herd weight distribution satisfies the abnormality determination condition. Examples of the abnormality determination condition include a case where there is an individual whose estimated weight does not change for a certain period of time and a case where there is an individual showing a tend different from the overall trend of the herd weight distribution among so-called outliers in which the difference from the average estimated weight is equal to or greater than a predetermined value in the herd weight distribution. The alert information generated when the herd weight distribution satisfies such an abnormality determination condition includes, for example, notification information indicating that the pig pen having a herd weight distribution satisfying the abnormality determination condition contains a pig with abnormality such as disease, injury, and death.

The livestock information management unit 118 can generate alert information also when the livestock image information acquisition unit 112 cannot acquire the livestock image information normally or the feeding information acquisition unit 114 cannot acquire feeding information normally. The case where the livestock image information or the feeding information cannot be acquired normally includes a case where livestock image information or feeding information cannot be acquired for a predetermined period of time or continuously for a predetermined number of times from the barn 101, a case where the acquired livestock image information or feeding information is corrupted, and the like. The alert information generated in such cases includes, for example, notification information indicating that the camera 102 or the feeder 103 in the target pig pen may have a failure.

The livestock information management unit 118 can cause the generated livestock management information and alert information to be stored in a rearing management DB 117 and transmit the livestock management information as shown in FIG. 7 in response to a request from the information terminal 201 or at a regular basis. The livestock information management unit 118 transmits alert information to the information terminal 201 immediately after generation of the alert information.

The information terminal 201 is, for example, a device such as a PC, a smartphone, a tablet PC, and a mobile phone. The information terminal 201 may access the livestock information management server 111 via dedicated application software installed in the information terminal 201. Alternatively, the information terminal 201 may access the livestock information management server 111 by using an operating environment (e.g., an application programming interface (API) and a platform) provided by the livestock information management server 111.

The input unit 211 has a function of being operated by a user to input or select information, such as a keyboard, a mouse, or a touch pad. Alternatively, a touch panel integrated with the display unit 212 such as a liquid crystal display or an organic EL display in a smartphone, a tablet, or a PC may be used. The input unit 211 may also be a voice input unit.

The display unit 212 is a display device or the like having a function of displaying information or the like to a user. The information terminal 201 may be an independent display device, or a display device such as a liquid crystal display and an organic EL display in a smartphone and a tablet. The display unit 212 can also display the herd weight distribution and the feeding information received from the livestock information management server 111 and associated with each other and the trend information as shown in FIG. 7.

The information terminal 201 may be integrated with the livestock information management server 111 without using the network NW

### <Process Flow>

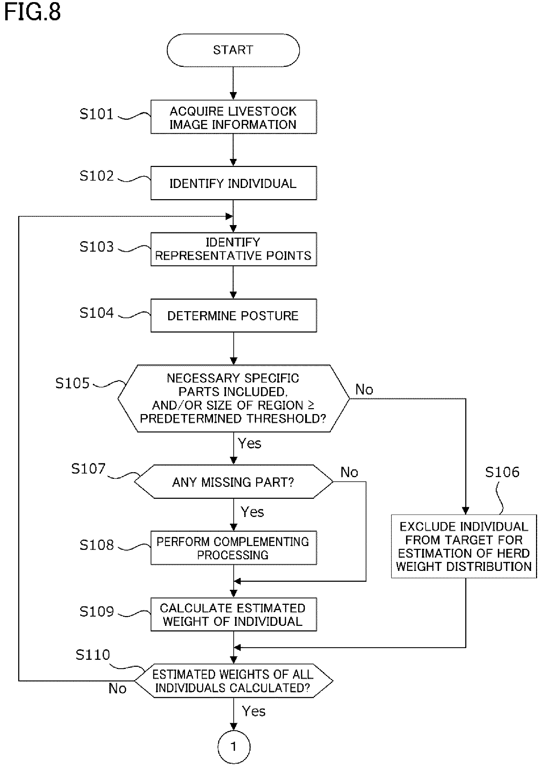

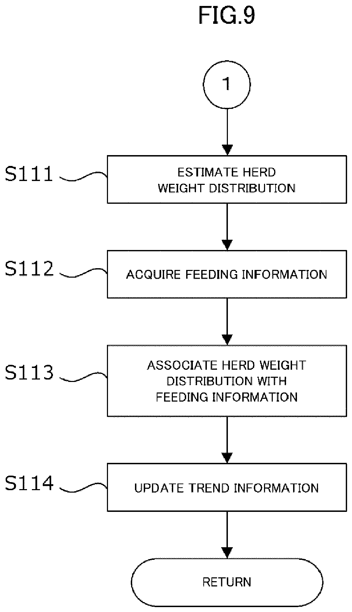

FIGS. 8 and 9 show flowcharts of a livestock management information generation routine executed in the livestock information management server 111 of the livestock information management system 1 according to the embodiment of the present disclosure. The following describes a livestock information management method according to the flowcharts. The flowchart is a mere example, and the livestock management information generation routine is not limited to the process of the flowchart.

In step S101 of FIG. 8, the herd weight estimation unit 116 of the livestock information management server 111 acquires livestock image information from the livestock image information acquisition unit 112 or the livestock image DB 113 (livestock image information acquisition), and the process proceeds to step S102.

In step S102, the herd weight estimation unit 116 identifies individual pigs from the livestock image of the acquired livestock image information by image processing, and the process proceeds to step S103.

In step S103, the herd weight estimation unit 116 recognizes representative points of parts in each of the identified individuals, and the process proceeds to step S104.

In step S104, the herd weight estimation unit 116 determines a posture of each individual from arrangement of the representative points, and the process proceeds to step S105. When the information on the arrangement of the representative points is used as posture information, step S104 may be omitted.

In step S105, the herd weight estimation unit 116 determines whether the identified representative points include specific parts necessary for estimated weight calculation and/or the size (the area or the number of pixels) of a region of each individual on the livestock image is equal to or greater than the predetermined threshold. If the determination result is false (No), i.e., the representative points do not include the specific parts necessary for estimated weight calculation, and/or the size of the region of the individual is less than the predetermined threshold, the process proceeds to step S106.

In step S106, the herd weight estimation unit 116 excludes the individual identified in step S102 from a target for herd weight distribution estimation.

On the other hand, if the determination result in step S105 is true (Yes), i.e., the representative points include the specific parts necessary for estimated weight calculation, and/or the size of the region of the individual is equal to or higher than the predetermined threshold, the process proceeds to step S107.

In step S107, the herd weight estimation unit 116 determines whether or not the region of the individual identified in step S102 has a missing part for an outline of pig. If the determination result is true (Yes), i.e., the region of the identified individual has the missing part, the process proceeds to step S108. If the determination result is false (No), i.e., the region of the identified individual does not have the missing part, the process skips step S108 and proceeds to step S109.

In step S108, the herd weight estimation unit 116 complements the missing part by using a part-specific template corresponding to the missing part, and the process proceeds to step S109.

In step S109, the herd weight estimation unit 116 calculates the estimated weight corresponding to the region of the individual, and the process proceeds to step S110.

In step S110, the herd weight estimation unit 116 determines whether or not the estimated weights of all individuals identified in the livestock image information acquired in step S101 have been calculated. If the determination result is false (No), i.e., there is an individual for which the estimated weight has not been calculated yet, the process returns to step S103, and representative points for next individual are identified, and the steps mentioned above are repeated thereafter. On the other hand, if the determination results in step S110 is true (Yes), i.e., calculation of the estimated weights of all individuals has been completed, the process proceeds to step S111 of FIG. 9.

In step S111 of FIG. 9, the herd weight estimation unit 116 performs statistical processing on the estimated weights of all individuals, and estimates the herd weight distribution (herd weight estimation) and the process proceeds to step S112.

In step S112, the livestock information management unit 118 of the livestock information management server 111 acquires feeding information corresponding to the herd weight distribution estimated in step S111 from the feeding information acquisition unit 114 or the livestock image DB 115 (feeding information acquisition), and the process proceeds to step S113.

In step S113, the livestock information management unit 118 generates livestock management information by associating the herd weight distribution estimated in step S111 with the feeding information acquired in step S112 (livestock information management), and the process proceeds to step S114.

In step S114, the livestock information management unit 118 updates trend information along a time series of the herd weight distribution and the feeding information associated with the herd weight distribution, and the routine returns. The herd weight distribution and the feeding information associated with each other in step S113 and the trend information updated in step S114 are stored in the rearing management DB 117.

The livestock information management server 111 performs, at a regular basis, the livestock management information generation routine as mentioned above to update the livestock management information in the rearing management DB 117, and the farmer can browse the updated livestock management information via the information terminal 201.

As described above, in the livestock information management system 1, the herd weight distribution of a plurality of livestock is estimated from the livestock image information, and the estimated herd weight distribution is associated with the feeding information to generate the livestock management information. The herd weight distribution is estimated from the livestock image information. Thus, it is not necessary to measure weights of individual pigs one by one, and the weight distribution in the herd can be easily estimated while reducing the facility costs and burdens on the farmers and pigs. The herd weight distribution estimated as described above is then associated with the feeding information. This facilitates recognition of the relationship between the growth of pigs and feed.

According to the present embodiment, the weights and feed for the livestock can be easily managed even in a large-scale rearing farm with a small number of workers while reducing a cost increase.

Further, trend information along a time series of the herd weight distribution and the feeding information associated with the herd weight distribution is generated. This further facilitates recognition of the relationship between the growth of pigs and feed. Accordingly, the farmer can easily and accurately optimize the contents of feed, and the cost of rearing pigs can be reduced.

The feeding information includes at least one of a feeding timing, the type of feed, a feeding amount, a nutrient, an additive, a water supply timing, or a water supply amount. This allows clarification of the relationship between the feeding information and the herd weight distribution, and optimization of the contents of feed by the farmer.

The herd weight estimation unit 116 identifies the region indicating each individual pig in the livestock image information, calculates the estimated weight corresponding to the region of each individual, and estimates the herd weight distribution from the estimated weight. As described above, the herd weight distribution is estimated from the estimated weight of each individual in the livestock image information. This allows accurate estimation of the herd weight distribution.

The herd weight estimation unit 116 identifies representative points corresponding predetermined parts of each individual, and excludes an individual including no representative point (specific part) necessary for weight estimation and an individual where a value (an area or the number of pixels) correlated with the size of the region of the individual is less than the predetermined threshold, from a target for herd weight distribution estimation. As described above, the individual whose estimated weight is difficult to be calculated accurately is excluded from the target for herd weight distribution estimation. This allows improvement in accuracy of the herd weight distribution.

On the other hand, for an individual including representative points (specific parts) necessary for weight estimation and an individual where the value (an area or the number of pixels) correlated with the size of the region of the individual is equal to or higher than the predetermined threshold, a missing part, if any, is complemented, and the estimated weight is then calculated. This allows further improvement in accuracy of the herd weight distribution.

The herd weight estimation unit 116 determines the posture of each individual identified, and calculates the estimated weight by using an estimation model corresponding to the posture. This allows reduction in error based on the posture and improvement in accuracy of the herd weight distribution.

When the herd weight distribution satisfies the abnormality determination condition, the livestock information management unit 118 generates alert information to alert the farmer. This allows an abnormal pig to be found in an early stage, and allows more efficient rearing to be realized.

Also when the livestock image information or the feeding information is not acquired normally, the livestock information management unit 118 generates alert information. This allows failures of devices in the barn 101 to be found in an early stage, and allows more efficient rearing to be realized.

### <Program>

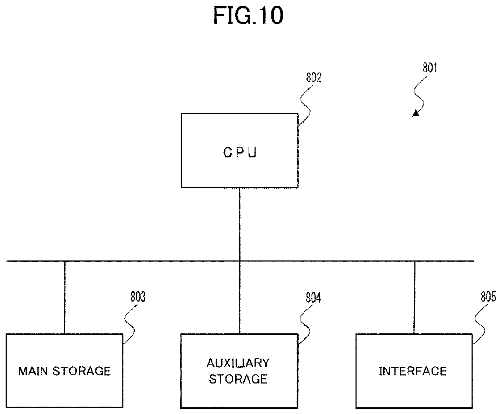

FIG. 10 is a schematic block diagram showing the configuration of a computer 801. The computer 801 includes a CPU 802, a main storage 803, an auxiliary storage 804, and an interface 805. The CPU 802 may be a GPU.

Here, a program for realizing each function constituting the livestock information management server 111 according to the embodiment will be described in detail.

The livestock information management server 111 is mounted on the computer 801. The operation of each component of the livestock information management server 111 is stored in the auxiliary storage 804 in a form of a program. The CPU 802 reads the program from the auxiliary storage 804, expands the program to the main storage 803, and executes the processes according to the program. In addition, the CPU 802 allocates storage areas corresponding to the above-described storage units in the main storage 803 according to the program.

The program specifically causes the computer 801 to execute: acquiring, by a livestock image information acquisition unit, livestock image information including a plurality of livestock belonging to a management herd; acquiring, by a feeding information acquisition unit, feeding information on feed for the livestock in the management herd; estimating, by a herd weight estimation unit, a herd weight distribution of the plurality of livestock from the livestock image information; and generating, by a livestock information management unit, livestock management information by associating the herd weight distribution estimated in the estimating of the herd weight with the feeding information.

The auxiliary storage 804 is an example of a non-transitory tangible medium. Other examples of the non-transitory tangible medium include magnetic disks, magneto-optical disks, CD-ROMs, DVD-ROMs, and semiconductor memories, to be connected via the interface 805. Alternatively, if the program is distributed to the computer 801 via the network NW, the computer 801 receiving the distribution may expand the program to the main storage 803, and execute the processes.

The program may realize some of the above-described functions. The program may also be a so-called differential file (differential program) that realizes the above-described functions in combination with other programs which have been stored in the auxiliary storage 804.

Although some embodiments of the present disclosure have been described above, these embodiments can be implemented in various other forms, and various omissions, substitutions, and changes can be made without departing from the spirit of the invention. These embodiments and variations thereof are included in the scope and spirit of the invention, and are also included in the invention described in the claims and the equivalent scope thereof. The embodiments have been described above with reference to an example where pigs for which the effects of the present disclosure are exhibited more efficiently than other livestock because the number of pigs reared in one farm is thousands to tens of thousands. However, the livestock information management system 1 can be applied to other livestock such as cattle and poultry. In this case, the estimation model can be appropriately set according to the type of livestock.

In the embodiment, the cameras 102a and 102b moving on the rails 106a and 106b provided above each pig pen 105 capture livestock images. However, the installation method of the cameras and the direction in which the livestock images are captured are not limited thereto. For example, the livestock image may be captured from the side by a camera installed on a wall of each pig pen, or may be captured in multiple directions.

### DESCRIPTION OF REFERENCE CHARACTERS

- 1: Livestock Information Management System
- 101: Barn
- 102: Camera
- 103: Feeder
- 104: Environment Status Acquisition Device
- 105: Pig Pen
- 111: Livestock Information Management Server
- 112: Livestock Image Information Acquisition Unit
- 113: Livestock Image Database
- 114: Feeding Information Acquisition Unit
- 115: Feeding Database
- 116: Herd Weight Estimation Unit
- 117: Rearing Management Database
- 118: Livestock Information Management Unit
- 201: Information Terminal
- 211: Input Unit
- 212: Display Unit
- 801: Computer
- 802: CPU
- 803: Main Storage
- 804: Auxiliary Storage
- 805: Interface

## Claims

1. A livestock information management system, **characterized by** comprising:
a livestock image information acquisition unit configured to acquire livestock image information including a plurality of livestock;
a feeding information acquisition unit configured to acquire feeding information on feed for the livestock;
a herd weight estimation unit configured to estimate a herd weight distribution of the plurality of livestock from the livestock image information; and
a livestock information management unit configured to generate livestock management information by associating the herd weight distribution estimated by the herd weight estimation unit with the feeding information.

2. The livestock information management system of claim 1, wherein
the livestock information management unit generates the livestock management information by associating the herd weight distribution with the feeding information so that an imaging timing for the livestock image information on which the herd weight distribution is based corresponds to a feeding timing in the feeding information.

3. The livestock information management system of claim 1 or 2, wherein
the livestock information management unit is configured to generate trend information along a time series of the herd weight distribution and the feeding information associated with the herd weight distribution.

4. The livestock information management system of any one of claims 1 to 3, wherein
the feeding information includes at least one of a feeding timing, a type of feed, a feeding amount, a nutrient, an additive, a water supply timing, or a water supply amount.

5. The livestock information management system of any one of claims 1 to 4, wherein
the herd weight estimation unit identifies regions indicating individuals of the livestock in the livestock image information, calculates estimated weights corresponding to the regions of the individuals, and estimates the herd weight distribution from the estimated weights.

6. The livestock information management system of claim 5, wherein
the herd weight estimation unit identifies predetermined parts of the livestock from the regions of the individuals identified, and excludes an individual which does not include one or more specific parts necessary for weight estimation among the parts identified, from a target for estimation of the herd weight distribution.

7. The livestock information management system of claim 5 or 6, wherein
the herd weight estimation unit identifies predetermined parts of the livestock from the regions of the individuals identified, and calculates an estimated weight of an individual including one or more specific parts necessary for weight estimation among the parts identified by complementing a missing part, if any, for an outline of the livestock in the region of the individual identified.

8. The livestock information management system of claim 5 or 6, wherein
the herd weight estimation unit excludes an individual where a value correlated with a size of the region of the individual identified is less than a predetermined threshold, from a target for estimation of the herd weight distribution.

9. The livestock information management system of claim 5 or 6, wherein
for an individual where a value correlated with a size of the region of the individual identified is equal to or higher than a predetermined threshold, the herd weight estimation unit calculates an estimated weight of the individual by complementing a missing part, if any, for an outline of the livestock in the region of the individual identified.

10. The livestock information management system of any one of claims 5 to 9, wherein
the herd weight estimation unit determines postures of the individuals identified and calculates estimated weights of the individuals by using an estimation model corresponding to each of the postures.

11. The livestock information management system of any one of claims 1 to 10, wherein
the livestock information management unit has an abnormality determination condition for determining an abnormal individual from the herd weight distribution, and generates alert information when the herd weight distribution estimated by the herd weight estimation unit satisfies the abnormality determination condition.

12. The livestock information management system of any one of claims 1 to 11, wherein
the livestock information management unit generates alert information when the livestock image information acquisition unit cannot acquire the livestock image information normally or the feeding information acquisition unit cannot acquire the feeding information normally.

13. A livestock information management server, **characterized by** comprising:
a livestock image information acquisition unit configured to acquire livestock image information including a plurality of livestock;
a feeding information acquisition unit configured to acquire feeding information on feed for the livestock;
a herd weight estimation unit configured to estimate a herd weight distribution of the plurality of livestock from the livestock image information; and
a livestock information management unit configured to generate livestock management information by associating the herd weight distribution estimated by the herd weight estimation unit with the feeding information.

14. A livestock information management method, **characterized by** comprising:
acquiring, by a livestock image information acquisition unit, livestock image information including a plurality of livestock;
acquiring, by a feeding information acquisition unit, feeding information on feed for the livestock;
estimating, by a herd weight estimation unit, a herd weight distribution of the plurality of livestock from the livestock image information; and
generating, by a livestock information management unit, livestock management information by associating the herd weight distribution estimated in the estimating of the herd weight distribution with the feeding information.

15. A livestock information management program, **characterized by** causing a computer to execute:
acquiring, by a livestock image information acquisition unit, livestock image information including a plurality of livestock;
acquiring, by a feeding information acquisition unit, feeding information on feed for the livestock;
estimating, by a herd weight estimation unit, a herd weight distribution of the plurality of livestock from the livestock image information; and
generating, by a livestock information management unit, livestock management information by associating the herd weight distribution estimated in the estimating of the herd weight distribution with the feeding information.
